## Europäisches Patentamt
## European Patent Office
## Office européen des brevets

(19)

(11) Numéro de publication: **0 139 674**
**B1**

(12)

# FASCICULE DE BREVET EUROPÉEN

(45) Date de publication du fascicule du brevet:
**04.11.87**

(51) Int. Cl.⁴: **A 01 H 1/02, C 12 N 15/00** .

(21) Numéro de dépôt: **84901088.9**

(22) Date de dépôt: **16.03.84**

(86) Numéro de dépôt international:
**PCT/FR 84/00065**

(87) Numéro de publication internationale:
**WO 84/03606 (27.09.84 Gazette 84/23)**

(54) **PROCEDE D'HYBRIDATION SOMATIQUE DE COLZA.**

(30) Priorité: **16.03.83 FR 8304298**

(43) Date de publication de la demande:
**08.05.85 Bulletin 85/19**

(45) Mention de la délivrance du brevet:
**04.11.87 Bulletin 87/45**

(84) Etats contractants désignés:
**AT BE CH DE GB LI LU NL SE**

(56) Documents cité:
**US-A-3 570 181**
**US-A-3 832 801**

**Fette, Seifen, Anstrichmettel, volume 78, février
1976, S. S. Radwan "Tissue culture, a new tool for
propagating and breeding rape and other plants",
pages 70-76**

(73) Titulaire: **INSTITUT NATIONAL DE LA
RECHERCHE AGRONOMIQUE (INRA), 145, Rue
de l'Université, F-75341 Paris Cedex 07 (FR)**

(72) Inventeur: **PELLETIER, Georges, 28, avenue de
l'Espérance, F-91440 Bures- sur- Yvette (FR)**

(74) Mandataire: **Warcoin, Jacques, Cabinet Régimbeau
26, avenue Kléber, F-75116 Paris (FR)**

**0 139 674**

## Description

La présente invention concerne notamment un procédé d'hybridation somatique permettant la création de nouvelles variétés de colza.

Une augmentation de rendement du colza pourrait constituer un élément permettant de réduire le déficit des pays de la C.E.E. en oléoprotéagineux.

Or, si le colza, espèce bien adaptée aux climats européens, a déjà bénéficié d'amélioration génétiques importantes (introduction de résistances aux maladies, amélioration de la qualité de l'huile et des tourteaux), les variétés cultivées en France sont des lignées pures. On pourrait donc espérer une augmentation de rendement appréciable (de l'ordre de 20 %) en créant des variétés hybrides F1. Cette création implique que l'on puisse disposer d'un système de stérilité mâle cytoplasmique permettant la "castration génétique" de la lignée servant de parent femelle pour ces hybrides. ·

On n'a pas trouvé dans l'espèce colza (Brassica napus) de géniteurs mâle-stériles cytoplasmiques exploitables. En revanche, une stérilité mâle cytoplasmique stable a pu être obtenue en transférant le cytoplasme du radis au colza par des croisements adéquats. Malheureusement, ce transfert de cytoplasme a également conféré au colza une déficience chlorophyllienne thermosensible qui rend inutilisable telle quelle la lignée mâle-stérile obtenue.

En effet, à partir de 12°C on observe un jaunissement des feuilles, en particulier pendant la première période de végétation, ce qui accroît la sensibilité de la plante au froid pendant une période délicate de végétation.

Le Demandeur a pu mettre au point un procédé permettant d'obtenir des colza présentant une stérilité mâle cytoplasmique (smc) et présentant des caractères cytoplasmiques déterminés portés par un parent fertile, notamment une non déficience chlorophyllienne au froid.

Il s'agit d'un procédé d'obtention de colza mâle stérile possédant un caractère cytoplasmique déterminé, caractérisé en ce que:

- on effectue la fusion somatique des protoplastes de deux colza, A et B,

A présentant une stérilité mâle, et

B, fertile, présentant ledit caractère cytoplasmique,

et

- on cultive les produits de fusion jusqu'à l'obtention de plantules.

Les cybrides (cytoplasme hybride) obtenus sont des lignées smc qui, par exemple, ne présentent pas de jaunissement lorsque la température descend au-dessous de 12°C.

Le parent A peut être notamment un colza sur cytoplasme de radis japonais smc, ou un colza smc, lesquels présentent une déficience chlorophyllienne à basse température.

Le parent B peut être notamment un colza fertile sans déficience chlorophyllienne qui présente éventuellement un caractère cytoplasmique intéressant, par exemple une résistance aux triazines (colza sur cytoplasme de navette canadienne résistante aux triazines).

Ce dernier caractère permet d'envisager un désherbage des colza grâce aux triazines.

Bien que l'on puisse envisager diverses origines pour les protoplastes (le protoplaste est une cellule végétale débarrassée de sa paroi pectocellulosique mécaniquement ou par digestion enzymatique), on utilisera de préférence des protoplastes isolés de tissus de jeunes feuilles cultivées in vitro.

La fusion des protoplastes peut être mise en oeuvre selon des processus connus, en particulier selon la méthode décrite par Chupeau et al.

Les produits de fusion sont cultivés sur un milieu nutritif contenant des substances de croissance, notamment des auxines, en particulier l'acide alphanaphtalène acétique (ANA), l'acide 2,4-dichloro-phénoxyacétique (2,4 D) ou d'autres auxines, en combinaison avec des cytokinines telles que la benzylaminopurine (BA).

Le rapport cytokinines/auxines est en général voisin de 1 et le rapport auxine/2,4 D est compris entre 0,1 et 1.

Le rapport pondéral de ces produits peut être de ANA:BA: 2,4 D, 0,5:0,5:0,5 et en général de 1:1:0,25.

Dans le premier milieu de culture des produits de fusion, on utilisera de préférence comme source de carbone le glucose, l'agent osmotique étant le mannitol.

Ce premier milieu de culture contient évidemment les différents éléments d'un milieu nutritif notamment:

- des macroéléments

. dérivés azotés en particulier $KNO_3$ et $(NH_4)_2SO_4$

. des phosphates tels que $NaH_2PO_4$

. du calcium, $CaCl_2$ et

. du magnésium, $MgSO_4$;

- des oligoéléments, notamment du Fe sous forme d'éthylène diamine tétracétate de Fe;

- ainsi que des vitamines et éventuellement des acides aminés.

Il s'agit là de composants pour des milieux de culture connus, certaines précisions sur les dosages et la nature exacte de ces composants apparaîtront à la lecture des exemples particuliers.

Afin d'assurer une culture continue des produits de fusion, on doit effectuer périodiquement des dilutions du milieu initial (appelé parfois milieu de callogénèse) avec des milieux de culture frais qui accélèrent la croissance des cals.

Ces milieux nutritifs contiendront, de préférence, à la place de glucose, du saccharose, et la teneur en agent osmotique (mannitol) ira en décroissant.

2

Ces dilutions seront effectuées, de préférence, après 12 à 14 jours et 17 à 22 jours de culture sur le milieu initial.

Après 29 à 36 jours, les colonies sont de préférence placées sur un milieu nutritif solide à l'agar.

Les bourgeons qui se développent sont repiqués sur un milieu solide frais entre 2 et 8 semaines.

Lorsque des méristèmes bien développés apparaissent, les bourgeons sont bouturés sur milieu nutritif.

Les boutures donnent naissance à des plants de colza parmi lesquels on sélectionne les plants ayant la caractéristique smc et présentant l'un des marqueurs cytoplasmiques du parent fertile.

Des caractéristiques plus précises des différents milieux seront mentionnées dans les exemples.

Les exemples suivants sont destinés à illustrer d'autres caractéristiques et avantages de la présente invention sans pour autant la limiter.

Ces exemples mentionnent différents milieux de culture qui sont identifiés par des lettres et dont les compositions sont rassemblées dans le tableau suivant les exemples.

## Exemple 1

Préparation et fusion des protoplastes de colza

On met en oeuvre les parents suivants:

Le premier parent A est un colza de printemps sur cytoplasme radis japonais.

Il possède les caractères suivants: Caractères nucléaires

a) fleurs avec pétales larges

b) présence d'acide érucique

Caractères cytoplasmiques

c) anthères non développées - absence de pollen (stérilité mâle)

d) nectaires non développés - très peu de nectar

e) plantes devenant vert clair à jaune aux températures inférieures à 12°C.

Le deuxième parent B est un colza de printemps, variété Brutor, possédant les caractères suivants:

Caractères nucléaires

a) fleurs avec pétales étroits

b) absence d'acide érucique

Marqueurs genétiques cytoplasmiques

c) anthères bien développées - présence de pollen

d) présence de nectaires - nectar abondant

e) pas de déficience chlorophyllienne au froid.

De plus, il est possible de distinguer les ADN chloroplastiques et mitochondriaux de ces deux parents par électrophorèse des fragments de restriction.

Ces colzas sont cultivés sous forme de plantules in vitro (2 000 lux, lumière blanche, 24°C). Elles sont obtenues à partir de germinations aseptiques (désinfection des graines dans l'hypochlorite de calcium pendant 20 minutes et rinçage avec de l'eau distillée stérile) sur le milieu F.

Après 8 jours, l'apex est isolé et mis en culture sur le même milieu. Les repiquages sont ensuite espacés de 4 semaines. On ne garde que le bourgeon apical pour le repiquage suivant.

Les protoplastes sont isolés des feuilles ainsi obtenues. Les feuilles ayant 3 semaines sont utilisées. Elles sont découpées en lanières de 2 à 3 mm de largeur, la nervure principale est retirée. Ces morceaux de feuilles sont placés dans des flacons contenant le mélange suivant:

- Milieu A

Pectolyase Y23, 0,1 %

Cellulase R10, 0,2 %

Les flacons sont agités pendant 16 heures (1 aller et retour toutes les 2 secondes, amplitude 5 cm) à l'obscurité.

Les protoplastes obtenus sont débarrassés des morceaux de feuilles non digérés par filtration sur tamis métallique de 40 microns de maille.

Le liquide est centrifugé à 700 g pendant 5 minutes. Le surnageant éliminé, les protoplastes sont rincés par deux centrifugations supplémentaires dans un milieu contenant 2,5 % KCl, 0,2 % $CaCl_2$, $2H_2O$, pH 6.

Les protoplastes sont fusionnés selon le procédé décrit par Chupeau et al.

## Exemple 2

Culture des protoplastes fusionnés

Les protoplastes obtenus à l'exemple 1 sont mis en culture à raison de 50 000/ml dans le milieu A et placés à l'obscurité à 28°C. Le milieu A présente notamment comme caractéristiques:

- de ne posséder comme source carbonée que le glucose,

- d'avoir comme agent osmotique le mannitol,

3

- un équilibre BA:ANA: 2,4 0 de 1:1:0,25;
- l'apport azoté provenant principalement de $KNO_3$.

Après 3 jours, les cultures sont mises à la lumière à la même température.

Après 12 à 14 jours de culture, le milieu A est additionné d'une quantité égale de milieu B et les suspensions sont réparties dans de nouvelles boîtes de culture (boîtes de Pétri en plastique de 100 mm de diamètre, chaque boîte contenant 10 ml de culture).

Cet apport de milieu frais a pour but d'accélérer le rythme de croissance des cals par une diminution de la pression osmotique et un apport énergétique sous forme de saccharose.

Après 17 à 22 jours de culture, les cultures sont diluées dans la proportion 1/1 par le milieu C et réparties dans de nouvelles boîtes de Pétri.

Après 29 à 36 jours, les colonies bien développées sont placées sur le milieu solide D à raison de 20 colonies par boîte contenant 25 ml de milieu.

Les bourgeons qui se développent entre la 2ème et la 8ème semaine sont isolés et mis en culture sur le milieu E. Quand de nouveaux méristèmes sont formés et bien développés sur ces bourgeons, ceux-ci sont isolés et bouturés sur le milieu F.

Le protocole expérimental permet d'obtenir une colonie pour 100 protoplastes mis en culture. Une plantule pour 10 colonies repiquées sur le milieu D sera ensuite obtenue soit au total un rendement de 1 plante pour 1000 protoplastes.

Les hybrides obtenus présentent les caractères cytoplasmiques suivants:

c) absence de pollen
d) présence de nectaires
e) pas de déficience chlorophyllienne au froid.

Ils possèdent le génome chloroplastique du colza et un génome mitochondrial hybride entre le colza et le radis.

Leurs descendances sexuées sont homogènes et conservent ces caractères.

**Exemple 3**

On opère comme dans les exemples 1 et 2 mais en mettant en oeuvre les parents suivants:

Le premier parent est toujours A.

Le second parent C est un colza de printemps (Tower) sur le cytoplasme d'une navette canadienne résistante aux triazines.

Il possède les caractères nucléaires suivants:

a) fleurs avec pétales étroits
b) absence d'acide érucique
et les caractères cytoplasmiques suivants:
c) présence de pollen
d) présence de nectaires
e) pas de déficience chlorophyllienne
f) résistance à l'atrazine.

Les génomes chloroplastiques et mitochondriaux sont différents de ceux du parent A et discernables par restriction.

On obtient des plantes hybrides qui possèdent les caractères cytoplasmiques suivants:

c) absence de pollen
d) présence de nectaires
e) pas de déficience chlorophyllienne
f) résistance à l'atrazine.

Elles possèdent les chloroplastes de la navette.

**Composition des milieux (mg/l)**

| | P | A | B | C | D | E | F |
|---|---|---|---|---|---|---|---|
| $NH_4NO_3$ | 1 650 | | 200 | 200 | 1 650 | 1 650 | 825 |
| $KNO_3$ | 1 900 | 2 500 | 1 250 | 1 250 | 1 900 | 1 900 | 950 |
| $(NH_4)_2SO_4$ | | 134 | 67 | 67 | | | |
| $NaH_2PO_4$ | | 150 | 75 | 75 | | | |
| $KH_2PO_4$ | 170 | | 35 | 35 | 170 | 170 | 85 |
| $CaCl_2,2H_2O$ | 440 | 750 | 525 | 525 | 440 | 440 | 220 |
| $MgSO_4,7H_2O$ | 370 | 250 | 250 | 250 | 370 | 370 | 185 |
| $H_3BO_3$ | 12,4 | 3 | 3 | 12,4 | 12,4 | 6,2 | 6,2 |
| $MnSO_4,4H_2O$ | 33,6 | 10 | 10 | 33,6 | 33,6 | 22,3 | 22,3 |
| $ZnSO_4,7H_2O$ | 21 | 2 | 2 | 21 | 21 | 8,6 | 8,6 |
| KI | 1,66 | 0,75 | 0,75 | 1,66 | 1,66 | 0,83 | 0,83 |
| $Na_2MoO_4,2H_2O$ | 0,5 | 0,25 | 0,25 | 0,5 | 0,5 | 0,25 | 0,25 |
| $CuSO_4,5H_2O$ | 0,05 | 0,025 | 0,025 | 0,05 | 0,05 | 0,025 | 0,025 |
| $CoCl_2,6H_2O$ | 0,05 | 0,025 | 0,025 | 0,05 | 0,05 | 0,025 | 0,025 |
| $FeSO_4,7H_2O$ | 22,24 | 27,8 | 27,8 | 27,8 | 27,8 | 27,8 | 22,24 |
| $Na_2EDTA$ | 29,84 | 37,3 | 37,3 | 37,3 | 37,3 | 37,3 | 29,84 |
| Inositol | 100 | 100 | 100 | 100 | 100 | 100 | 100 |
| Acide nicotinique | 0,5 | 1 | 1 | 1 | 0,5 | 1 | 0,5 |
| Pyridoxine HCl | 0,5 | 1 | 1 | 1 | 0,5 | 1 | 0,5 |
| Thiamine | | 10 | 10 | 10 | | 10 | |
| Glycine | 2 | | | | 2 | | 2 |
| Glucose | 10 000 | 20 000 | | | | | 10 000 |
| Saccharose | 10 000 | | 20 000 | 20 000 | 10 000 | 10 000 | |
| Mannitol | | 70 000 | 40 000 | | 20 000 | | |
| A.N.A. | | 1 | 0,2 | | 1 | 0,1 | 0,01 |
| B.A. | | 1 | 1 | | | 0,5 | |
| 2,4 D | | 0,25 | | 1 | | | |
| Sulfate d'adénine | | | | 30 | | | |
| I.P.A. | | | | | 1 | | |
| $GA_3$ | | | | | 0,02 | | |
| Tween 80 | | 10 | | | | | |
| Agar (pH 5,8) | 8 000 | | | | 8 000 | 8 000 | 8 000 |
| Gentamicine (sulfate) | 10 | | | | | | |

## Revendications

1. Procédé d'obtention de colza mâle stérile possédant un caractère cytoplasmique détérminé, caractérisé en ce que:
- on effectue la fusion somatique des protoplastes de deux colzas A et B,
  · A présentant une stérilité mâle, et
  · B, fertile, présentant ledit caractère cytoplasmique,
et
- on cultive les produits de fusion jusqu'à l'obtention de plantules.

2. Procédé selon la revendication 1, caractérisé en ce que les protoplastes sont isolés de tissus de jeunes feuilles cultivées in vitro.

3. Procédé selon l'une des revendications 1 et 2, caractérisé en ce que le milieu de culture des premiers produits de fusion, ou milieu de callogénèse, est un milieu de culture contenant, notamment, comme source de carbone du glucose, comme agent osmotique du mannitol, ayant un rapport cytokinine/auxine voisin de 1 et un rapport auxine/2,4 D compris entre 0,1 et 1.

4. Procédé selon la revendication 3, caractérisé en ce que le milieu de callogénèse primaire est dilué avec des milieux dans lesquels la source de carbone est le saccharose et dans lesquels la concentration en mannitol diminue.

5. Procédé selon l'une des revendications 1 à 4, caractérisé en ce que les colonies formées après dilution du milieu de callogénèse sont placées sur un milieu de culture solide à l'agar.

6. Procédé selon l'une des revendications 1 à 5, caractérisé en ce que le parent A est un colza sur cytoplasme de radis japonais smc ou un colza smc.

7. Procédé selon l'une des revendications 1 à 6, caractérisé en ce que le parent B est un colza fertile ne présentant pas de déficience chlorophyllienne et présentant éventuellement une résistance aux triazines.

**Patentansprüche**

1. Verfahren zum Herstellen von sterilem männlichen Raps, der eine bestimmte cytoplasmatische Eigenschaft aufweist,

dadurch gekennzeichnet, daß man die somatische Fusion der Protoplasten zweier Rapsarten A und B bewirkt,

wobei A eine männliche Sterilität aufweist und

B fruchtbar ist und die genannte cytoplasmatische Eigenschaft hat und

die Fusionsprodukte bis zum Erhalt von Keimen züchtet.

2. Verfahren nach Anspruch 1,

dadurch gekennzeichnet, daß die Protoplasten aus dem Gewebe in vitro gezüchteter junger Blätter isoliert werden.

3. Verfahren nach einem der Ansprüche 1 und 2,

dadurch gekennzeichnet, daß

das Nähr- oder Kallogenese-Medium der ersten Fusionsprodukte insbesondere Glukose als Kohlenstoffquelle und Mannit als osmotisches Mittel enthält und eine Verhältnis Cytokinin/Auxin von etwa 1 und ein Verhältnis von Auxin/2,4 D im Bereich von 0,1 bis 1 aufweist.

4. Verfahren nach Anspruch 3,

dadurch gekennzeichnet, daß

das primäre Kallogenese-Medium mit Medien verdünnt wird, deren Kohlenstoffquelle Saccharose ist und in denen die Mannit-Konzentration abnimmt.

5. Verfahren nach einem der Ansprüche 1 bis 4,

dadurch gekennzeichnet, daß

die nach dem Verdünnen des Kallogenese-Mediums gebildeten Kolonien auf ein festes Agar-Nährmedium übertragen werden.

6. Verfahren nach einem der Ansprüche 1 bis 5,

dadurch gekennzeichnet, daß die Elternzelle A ein Raps auf Cytoplasma von japanischem Rettich smc oder ein Raps smc ist.

7. Verfahren nach einem der Ansprüche 1 bis 6,

dadurch gekennzeichnet, daß die Elternzelle B ein fruchtbarer Raps ist, der keine Chlorophyllschwäche und gegebenenfalls eine Beständigkeit gegenüber Triazinen aufweist.


**Claims**

1. Process for obtaining sterile male colza possessing a prescribed cytoplasmic character, wherein:
- the somatic fusion of the protoplasts of two varieties of colza, A and B, is carried out,
· A having male sterility, and
· B, which is fertile, having the said cytoplasmic character,
and
- the fusion products are cultured until plantlings are obtained.

2. Process as claimed in claim 1, wherein the protoplast are isolated from tissues of young leaves cultured in vitro.

3. Process as claimed in one of claims 1 and 2, wherein the culture medium of the first fusion products, or callogenesis medium, is a culture medium containing, in particular, glucose as a source of carbon and mannitil as osmotic agent, and having a cytokinin/auxin ration in the region of 1 and an auxin/2,4-D ration between 0.1 and 1.

4. Process as claimed in claim 3, wherein the primary callogenesis medium is diluted with media in which the carbon source is sucrose and in which the concentration of mannitol decreases.

5. Process as claimed in one of claims 1 to 4, wherein the colonies formed after dilution of the callogenesis medium are placed on an agar-based solid culture medium.

6. Process as claimed in one of claims 1 to 5, wherein the parent A is a colza onto cms Japanese radish cytoplasm or a cms colza.

7. Process as claimed in one of claims 1 to 6, wherein the parent B is a fertile colza which does not have chlorophyll deficiency and optionally has resistance to triazines.